# EUROPEAN PATENT SPECIFICATION

(11) **EP 2 208 996 B1**
(45) Date of publication and mention of the grant of the patent: **01.09.2010**
(21) Application number: 09150740.0
(22) Date of filing: 16.01.2009
(51) Int. Cl.: G01N 33/49

(54) **A measuring unit for measuring characteristics of a sample liquid, in particular viscoelastic characteristics of a blood sample**
Messeinheit zum Messen der Eigenschaften einer Probenflüssigkeit, insbesondere der viskoelastischen Eigenschaften einer Blutprobe
Unité de mesure des caractéristiques d'un échantillon liquide, en particulier les propriétés visco-élastiques d'un échantillon sanguin

(43) Date of publication of application: 21.07.2010
(73) Proprietor: C A Casyso AG, 4132 Muttenz (CH)
(72) Inventor: Schubert, Axel, 81377, München (DE); Romero-Galeano, José Javier, 85570, Markt Schwaben (DE); Kessler, Max, 81539, München (DE)
(74) Representative: Peckmann, Ralf

(56) References cited:
- DE-A1- 2 740 932
- GB-A- 2 257 256
- US-A- 5 287 732
- US-A- 5 777 215

## Description

### FIELD OF THE INVENTION

The present invention relates to a measuring unit for measuring characteristics of a sample liquid, in particular viscoelastic characteristics of a blood sample. The present invention also relates to a corresponding measuring system.

### BACKGROUND

It is essential for survival that a wound stops bleeding, i.e. that the body possesses an adequate mechanism for haemostasis. The process of blood clotting can be activated in the case of injuries or inflammations by either extrinsic or intrinsic factors, e.g. tissue factor (TF) or Hagemann factor (F XII), respectively.

The other main constituent of the final blood clot are the thrombocytes which are interconnected by the fibrin fibres and undergo a number of physiological changes during the process of coagulation.

Various methods have been introduced to assess the potential of blood to form an adequate clot and to determine the blood clots stability. Common laboratory tests such as thrombocyte counts or the determination of fibrin concentration provide information on whether the tested component is available in sufficient amount but lack in answering the question whether the tested component works properly under physiological conditions (e.g. the polymerisation activity of fibrinogen under physiological conditions can not be assessed by common optical methods). Besides that, most laboratory tests work on blood-plasma and therefore require an additional step for preparation and additional time which is unfavourable especially under POC (point of care) conditions.

Another group of tests which overcomes these problems is summarized by the term "viscoelastic methods". The common feature of these methods is that the blood clot firmness (or other parameters dependent thereon) is continuously determined, from the formation of the first fibrin fibres until the dissolution of the blood clot by fibrinolysis. Blood clot firmness is a functional parameter, which is important for haemostasis in vivo, as a clot must resist blood pressure and shear stress at the site of vascular injury. Clot firmness results from multiple interlinked processes: coagulation activation, thrombin formation, fibrin formation and polymerization, platelet activation and fibrin-platelet interaction and can be compromised by fibrinolysis. Thus, by the use of viscoelastic monitoring all these mechanisms of the coagulation system can be assessed.

A common feature of all these methods used for coagulation diagnosis is that the blood clot is placed in the space between a cylindrical pin and an axially symmetric cup and the ability of the blood clot to couple those two bodies is determined.

The first viscoelastometric method was called "thrombelastography" (Hartert H: Blutgerinnungsstudien mit der Thrombelastographie, einem neuen Untersuchungsverfahren. Klin Wochenschrift 26:577-583, 1948). In the thromboelastography, the sample as a sample liquid is placed in a cup that is periodically rotated to the left and to the right by about 5°, respectively. A probe pin is freely suspended by a torsion wire. When a clot is formed it starts to transfer the movement of the cup to the probe pin against the reverse momentum of the torsion wire. The movement of the probe pin as a measure for the clot firmness is continuously recorded and plotted against time. For historical reasons the firmness is measured in millimetres.

A common feature of all these methods used for coagulation diagnosis is that the blood clot is placed in the space between a cylindrical pin and an axially symmetric cup and the ability of the blood clot to couple those two bodies is determined.

Calatzis et al. (US 5,777,215) describe a measuring illustrated in FIG. 1 which is known under the term thromboelastometry. Contrary to the modifications mentioned above, thromboelastometry is based on a cup 43 fixed in a cup holder 48 while a probe element 42 is actively rotated. For this purpose the probe element 42 is attached to a coupling shaft 35' which is suspended by a ball bearing 49 in a base plate 39 and has a drive element 32 connected to it. An oscillating motion perpendicular to the drawing plane induced at the opposite end of the drive element 32 is transformed into a periodically rotation of the coupling shaft 35' and the connected cup 43 around a rotation axis 20 by about 5° in each direction. As the sample liquid 44 begins to coagulate the motion amplitude of the coupling shaft 35' which is detected by e.g. the deflection of a light beam from detecting means 41 and a detecting element 31, e.g. a mirror, starts to decrease.

During coagulation the fibrin backbone creates a mechanical elastic linkage between the surfaces of the blood-containing cup 43 and the probe element 42 plunged therein. A proceeding coagulation process induced by adding one or more activating factor(s) can thus be observed. In this way, various deficiencies of a patient's haemostatic status can be revealed and can be interpreted for proper medical intervention.

A general advantage of viscoelastometric, e.g. thromboelastometric, techniques compared to other laboratory methods in this field therefore is that the coagulation process and the change of mechanical properties of the sample are monitored as a whole. This means that thromboelastometry does not only indicate if all components of the coagulation pathways are available in sufficient amounts but also if each component works properly.

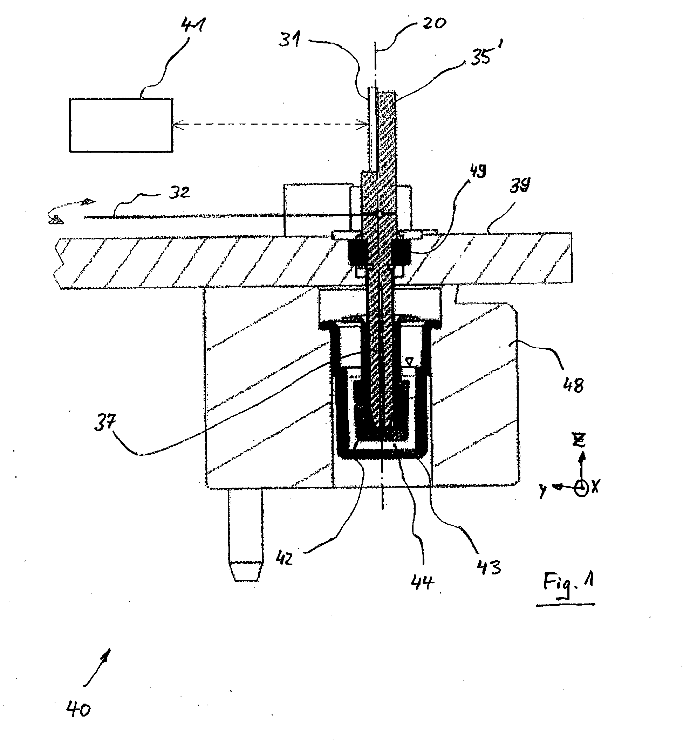

Compared with the device mentioned above using a torsion wire thromboelastometry as shown in FIG. 1 has the advantage that a ball bearing provides a certain stability of the measuring device, e.g. the measuring device can be configured as a transportable device and used under POC (point of care) conditions.

### SUMMARY OF THE INVENTION

It is a problem underlying the presented invention to provide an improved measuring unit for measuring characteristics of a sample liquid, in particular viscoelastic characteristics of a blood sample.

Directly connected to this invention is the problem to provide a corresponding improved measuring system for measuring viscoelastic characteristics of a sample liquid, in particular the coagulation characteristics of a blood sample liquid. These problems are solved by the subject-matter of the independent claims. Preferred embodiments are set forth in the dependent claims.

In a first aspect, the present invention provides measuring unit for measuring characteristics of a sample liquid, in particular viscoelastic characteristics of a blood sample, comprising a support member having at least one upper bearing arm with an upper bearing unit, at least one lower bearing arm with a lower bearing unit and a base for being attachable to a respective measuring system; a shaft having shaft toes and being rotatably supported about a rotation axis by said upper bearing unit and said lower bearing unit, wherein said upper bearing unit, said lower bearing unit and said shaft toes form toe bearings, respectively; an interface member having a detecting element and a drive element, said interface member being fixed on said shaft and being connected to a coupling shaft with a probe connector section for measuring characteristics of said sample liquid; wherein said interface member and the coupling shaft are coaxially aligned with said shaft.

In a second aspect, the present invention provides a measuring system for measuring characteristics of a sample liquid, in particular viscoelastic characteristics of a blood sample, comprising at least one measuring unit according to the invention.

Toe bearings have clearances and friction losses which are decreased in relation to ball bearings. In particular it is advantageous that toe bearings have smaller clearances regarding tilting as well. The measuring device according to the invention also has the advantage to provide a unit which does not need a basement being aligned horizontally with high precision. Thus it is highly suitable for use in POC conditions and the like. Moreover the measuring unit according to the invention is more unsusceptible in case of vibrations than that one of the state of the art. Another advantage is the miniaturized shape of toe bearings.

The support member forms a structural rigid component of the measuring unit and supports the shaft by the toe bearings. The shaft is coupled to a coupling shaft by an interface member being a component with several functions.

Said upper bearing unit and said lower bearing unit are removable inserts each being fixed in said respective bearing arm of said support member. Thus it is possible to remove the bearing units together with the shaft in case of maintenance without changing the support member.

Said lower bearing unit is a thrust bearing and said upper bearing unit is a moveable bearing. The moveable bearing is configured to be adjustable by adjusting means to achieve optimal friction and clearances.

The bearing units are equipped with bearing plates made of e.g. kind of jewel, e.g. sapphire or/and ceramic.

The interface member comprises an upper connector section connected to said shaft. This forms a simple connection to the shaft.

Furthermore said interface member has a frame having an opening for a passage of said lower bearing arm of said support member, which allows for a compact assembly of the measuring unit and a connection to the coupling shaft. Moreover a lower connector section for a connection to said coupling shaft forms another portion of the interface member.

Eventually the interface member has a front for securing that said detecting element thereon; and moreover a receptacle for securing that said drive element therein.

The interface member further comprises a fixing unit for fixing said interface member to said shaft. It is preferred that said fixing unit is at least one screw to achieve a simple fixing.

In an alternative embodiment said fixing unit is formed having clip means cooperating with corresponding clip means of said shaft.

It is preferred that said lower connector section is formed having clip means cooperating with corresponding clip means of an interface section of said coupling shaft for easy and fast connection without any tool.

It is also preferred that said probe connector section of said coupling shaft is formed having clip means cooperating with corresponding clip means of a probe element being detachably fixed onto said probe connector section. Thus an easy assembly and disassembly of different probe elements can be done.

In another preferred embodiment said detecting element can be a mirror.

When, in case of inserting the coupling shaft and/or a probe element onto the coupling shaft, an axial force can be exerted on the shaft and the interface member and cause an axial movement of the shaft in direction to the upper movable bearing. To limit said axial movement of the shaft and the interface member and a possible damage of the upper movable bearing the measuring unit can comprise axial stop means. Said stop means can be formed e.g. by a shoulder of the interface member or/and a shoulder of the shaft, said shoulder pointing to the upper bearing arm and co-operating with a corresponding shoulder of the upper bearing arm and/or the upper bearing unit.

In another embodiment at least one of said upper bearing unit and said lower bearing unit can comprises at least one bearing cover plate. Said bearing cover plate serves as a sealing and/or as a centre means for the shaft.

And in another embodiment it is preferred that the drive element is a spring wire.

### BRIEF DESCRIPTION OF THE DRAWINGS

Further features and advantages of the present invention will be evident from a description of embodiments with reference to the figures.

The figures are showing the following:
FIG. 1 is a schematic drawing of an example of thromboelastometry according to the state of the art.
FIG. 2 is a perspective exploded view of an exemplary embodiment of a measuring unit according to the invention.
FIG. 3 is a schematic view along y-direction of a sectional view along a rotation axis 20 of the assembled measuring unit of FIG. 1
FIG. 4 is a perspective view of an exemplary embodiment of a measuring device according to the invention.
FIG. 5 is a top view of the measuring device of FIG. 4.
FIG. 6 is a view along y-direction of the measuring device of FIG. 4.
FIG. 7 is a perspective view in x-direction of the measuring device of FIG. 4.

### DETAILED DESCRIPTION OF PREFERRED EMBODIMENTS

Parts and components having same functions are depicted with same references. Coordinate systems indicating x-, y- and z-direction in the figures allow for better orientation.

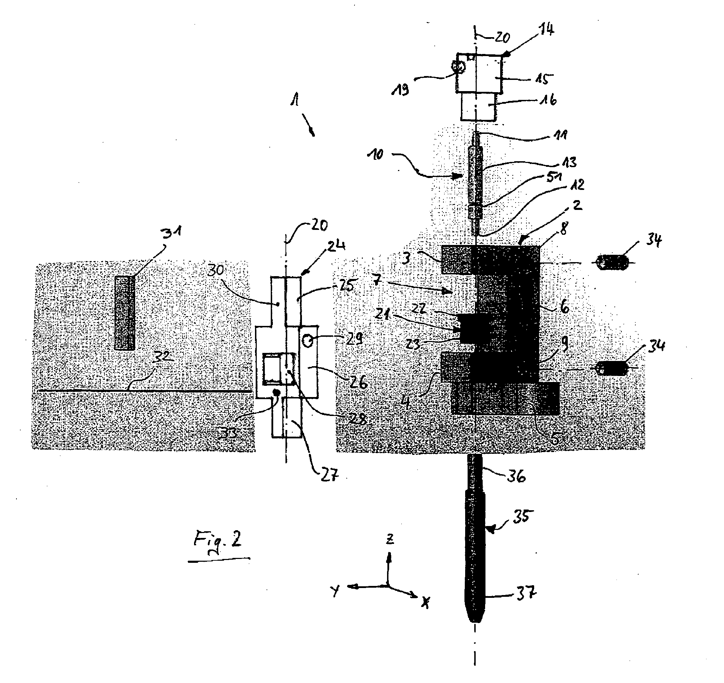

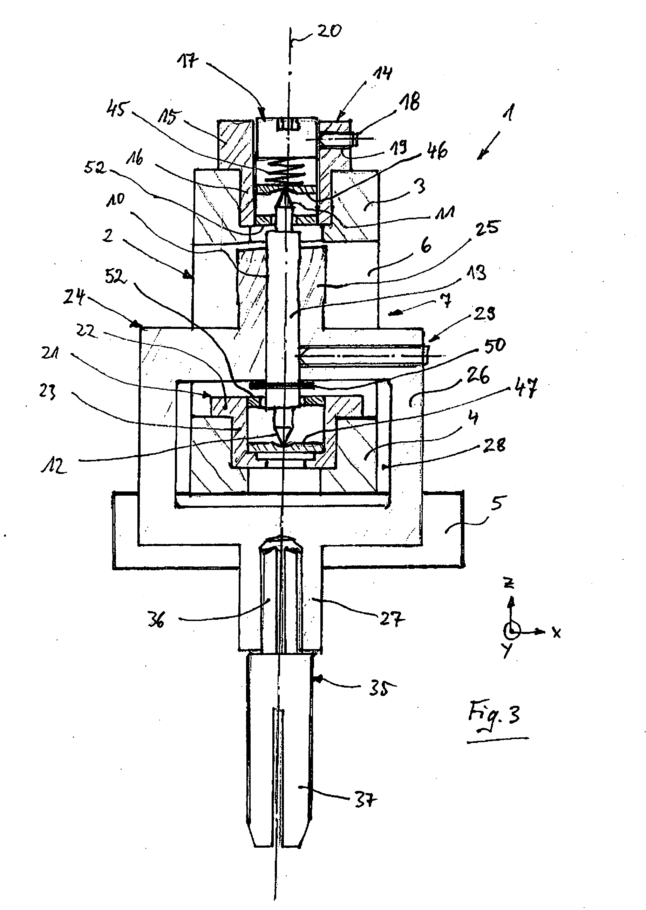

An exemplary embodiment of a measuring unit 1 according to the invention will now be described with regard to FIG. 2 and to FIG. 3.

FIG. 2 is a perspective exploded view of an exemplary embodiment of the measuring unit 1 according to the invention and FIG. 3 is a schematic view along y-direction of a sectional view along a rotation axis 20 of the assembled measuring unit 1 of FIG. 1.

The measuring unit comprises a support member 2 with a body 6 extending in z-direction. The body 6 connects an upper bearing arm 3 and a lower bearing arm 4. The bearing arms 3 and 4 extending in x-direction are parallel to each other spaced with a distance. Body 6 and said bearing arms 3 and 4 form a U-shaped block having with a recess 7 with the distance defined by the bearing arms 3 and 4.

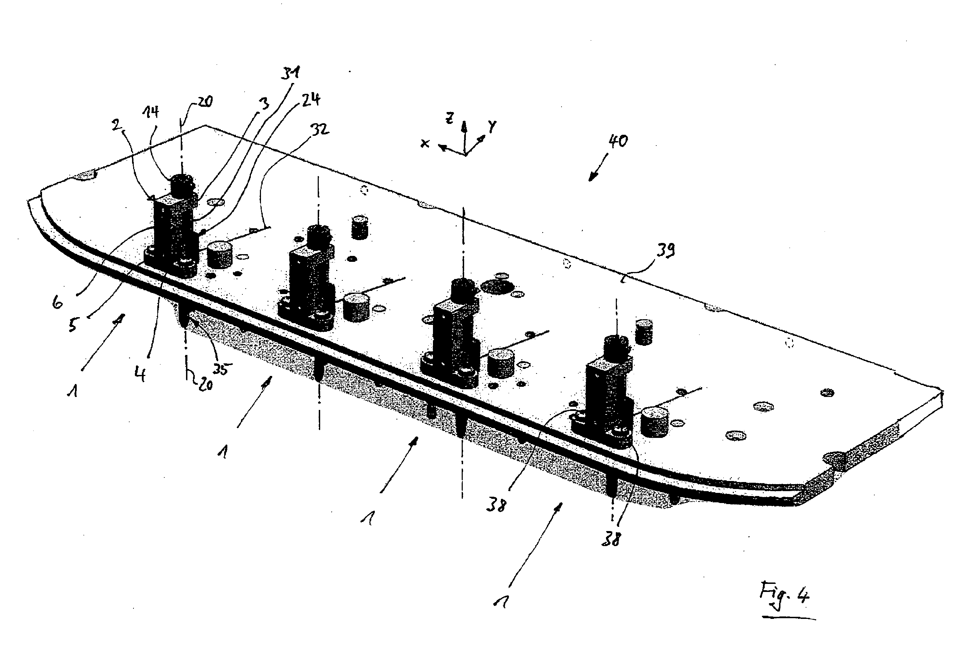

The body 6 and the lower bearing arm 4 are fixed onto a base 5 extending in x-direction. The base 5 comprises fixing holes (see FIG. 4 for mounting elements 38).

The upper bearing arm 3 is formed with a through hole for an upper bearing unit 14 and the lower bearing arm 4 has a through hole for a lower bearing unit 21, the through holes being aligned along a rotation axis 20.

The upper bearing unit 14 and the lower bearing unit 21 are formed as inserts to be inserted into said through holes. An upper fixing passage 8 extending from the side of the body 6 in the upper bearing arm 3 in y-direction allows for fixing the inserted upper bearing unit 14 by a fixing element 34, e.g. a screw. The inserted lower bearing unit 21 can be fixed similarly by a fixing element 34 via a lower fixing passage 9 extending in the lower bearing arm 4.

A shaft 10 with a shaft body 13 having an upper shaft toe 11 and a lower shaft toe 12 is provided to be supported by the bearing units 14 and 21. Said bearing units 14 and 21 are configured together with the shaft 10 having said upper shaft toe 11 and said lower shaft toe 12 to form a toe bearing, respectively. A groove 51 for a circlip 50 is formed in the shaft body 13.

The upper bearing unit 14 comprises a fixing section 15 and a bearing section 16. As can be seen from FIG. 3 the bearing section 16 is inserted into a through hole of the upper bearing arm 3. The upper bearing unit 14 is designed as a movable bearing with a movable bearing plate 46, e.g. made of a kind of jewel or ceramic, arranged in a through hole of the bearing unit 14. The upper shaft toe 11 of the shaft 10 rests on the movable bearing plate 46. The movable bearing plate 46 is movable along the rotation axis, i.e. in z-direction. A spring 45 is arranged in the fixing section 15 and can be adjusted by adjusting means 17, e.g. a screw, the adjusting means 17 being fixable by a fixing means 18, e.g. a screw through a fixing passage 19 in the fixing section 15.

The lower bearing unit 21 comprises a collar 22 and a bearing section 23. The bearing section 23 is inserted into a through hole of the lower bearing arm 4, wherein the collar 22 rests on the lower bearing arm 4. A thrust bearing plate 47 is arranged within a through hole of the lower bearing unit 21 and fixed therein. The trust bearing plate 47 is made of the material as the movable bearing plate, wherein the lower shaft toe 12 of the shaft 10 rests on the thrust bearing plate 47.

A pretension of the bearing units 14, 21 and the shaft 10 can be adjusted by the adjusting means 17.

The measuring unit 1 further comprises an interface member 24 having an upper connector section 25, a frame 26 and a lower connector section 27. An opening 28 is formed in the frame to form a passage having a cross-section greater than the cross-section of the lower bearing arm 4 (see also FIG. 3).

The upper connector section 25 is designed with a bore to be connected to the shaft 10 as can be seen from FIG. 3, wherein the shaft 10 is fixed by a fixing unit 29, e.g. a screw, to connect the interface member 24 to the shaft 10.

The lower connector section 27 is also provided with a bore to be connected to a coupling shaft 35. To this end the coupling shaft 35 is formed with an interface section 36 which is preferably designed as a clip means. Other fixing means can be possible. The coupling shaft 35 further comprises a probe connector section 37 which is provided to be connected to a probe element 42 (see FIG. 1).

Furthermore the interface member 24 has a front 30 on which a detecting element 31, e.g. a mirror (see also FIG. 1 and FIG. 2), is fixed, e.g. by glue. The interface member 24 also comprises a receptacle 33 on the side of the front at the lower portion of the frame 26. A drive element 32, e.g. a spring wire, as already shown in FIG. 1 can be inserted into this receptacle 33.

In this example the upper bearing unit 14 and the lower bearing unit 21 are equipped with bearing cover plates 52. Said bearing cover plates 52 are disc-shaped and inserted into the through holes of the bearing units 14 and 21 in such a way that they surround portions of the shaft 10 which extends through passage holes of the bearing cover plates 52. These passage holes have an inner diameter which is a little bit greater than that of the outer diameter of the corresponding shaft portion. So the bearing cover plates 52 provide a certain sealing function. On the other hand the bearing cover plates 52 can centre the shaft 10. In use there is no friction between the bearing cover plates 52 and the corresponding shaft portions. In the example of FIG. 3 the bearing cover plate 52 of the upper bearing unit 14 is inserted into the lowest part of the bearing section 16 and co-operates with a cylindrical portion of the upper shaft toe 11 of the shaft 10. The bearing cover plate 52 of the lower bearing unit 21 is inserted into the upper part of the collar 22 and co-operates with a cylindrical portion of the shaft body 13 of the shaft 10.

An assembly of the measuring unit 1 can be done as following to achieve the assembled unit (see FIG. 2 and 3).

First the lower bearing unit 21 is inserted into the lower bearing arm 4 and fixed as mentioned above. Then the interface member 24 is inserted into the recess 7 between upper bearing arm 3 and lower bearing arm 4 in such a way that the lower bearing 4 extends and protrudes through the opening 28 of the frame 26 of the interface member 24. The interface member 24 should be aligned with its bores to the rotation axis 20. Now the shaft 10 is inserted along the rotation axis 20 through the through hole in the upper bearing arm 3, through the bore of the upper connector section 25 of the interface member 24 into the lower bearing unit 21 so that the lower shaft toe 12 rests on the thrust plate 47 of the lower bearing unit.

Now the upper bearing unit 14 is inserted into the through hole of the upper bearing arm 3 and fixed as mentioned above, the movable bearing plate 46 resting on the upper shaft toe 11. Then the adjusting means 17 can be adjusted for a predefined pretension now or later.

The interface member 24 is shifted upwards on the shaft body 13 until the groove 51 can be fitted with a circlip 50. Then the interface member 24 is shifted downwards to rest on the circlip 50 to maintain a predefined axial position along the rotation axis 20. Now the interface member 24 can be fixed on the shaft body 13 by said fixing unit 29.

Thrust plate 47 and movable plate 46 comprise a dimple, respectively. The dimple will align the shaft 10 together with the interface member 24 and the coupling shaft 35.

Finally the coupling shaft 35, the detecting element 31 and the drive element 32 can be connected to the interface member 24.

When, in case of inserting the coupling shaft 35 and/or a probe element 42 (see FIG. 1) onto the coupling shaft 35, an axial force can be exerted via the interface member 24 on the shaft 10 and cause an axial movement of the shaft 10 in direction of the rotation axis 20 to the upper movable bearing 14. To limit said axial movement of the shaft 10 and the interface member 24 and a possible damage of the upper movable bearing 14 the measuring unit 1 can comprise axial stop means. Said stop means can be formed in the shown embodiment e.g. by an upper shoulder of the interface member 24 or/and a shoulder of the shaft 10, said shoulder pointing to the upper bearing arm 3 and co-operating with a corresponding shoulder of the upper bearing arm 3 and/or the upper bearing unit 14.

The assembled measuring unit 1 is shown in FIG. 3 and can be mounted on a base plate 39 of a measuring device 40 as shown in FIG. 4, which is a perspective view of an exemplary embodiment of a measuring device 40 according to the invention.

In the shown embodiment the measuring device 40 comprises four measuring units 1. The measuring units 1 are fixed on the base plate 39 by mounting elements, e.g. screws or clip fixing means, the coupling shafts 35 extending through corresponding openings in the base plate 39. The drive elements 32 extend in y-direction and can be driven by a not shown driving device to oscillate the respective interface members 24 with the thereto connected respective coupling shafts 35 (and corresponding parts as mentioned above) around the respective rotation axis 20.

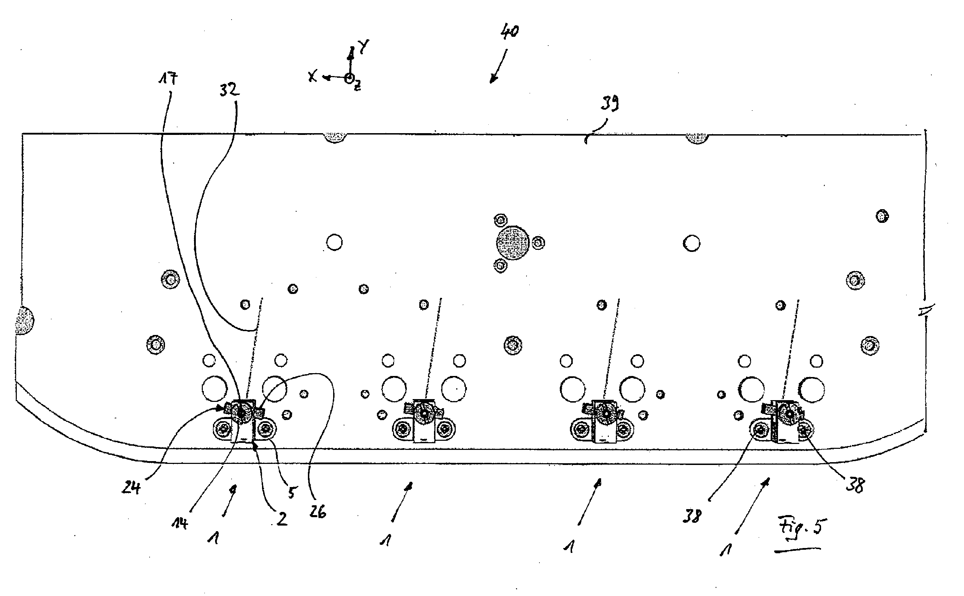

FIG. 5 is a top view of the measuring device 40 of FIG. 4. Here it is shown that the drive elements 32 and the connected interface members 24 are arranged in a specific angle to the y-direction.

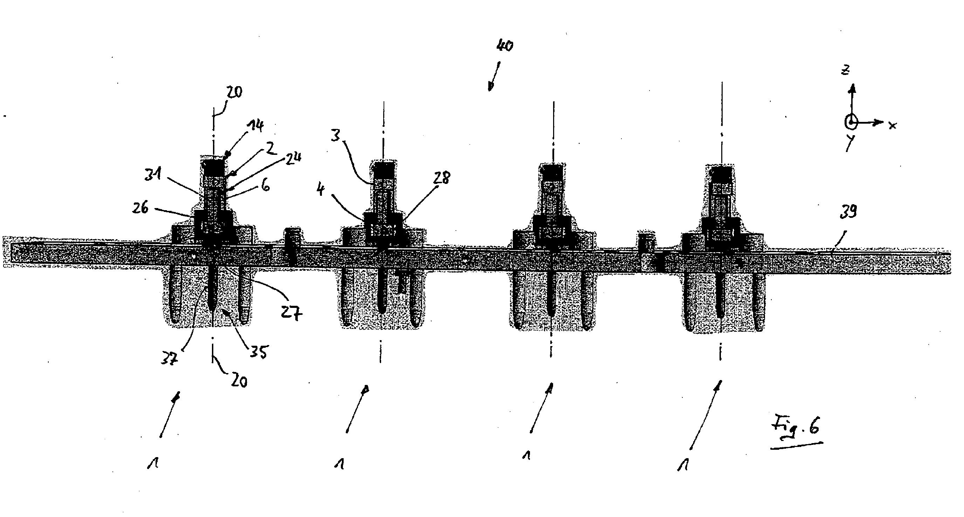

FIG. 6 is a view along y-direction of the measuring device of FIG. 4, wherein the detecting elements 31 can be seen from a front view.

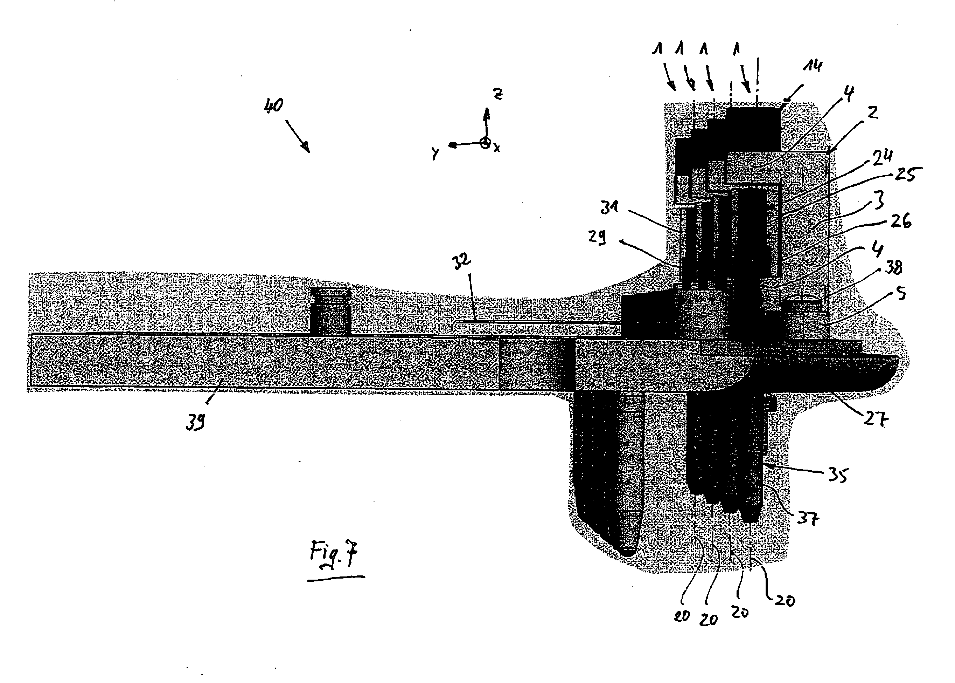

Finally FIG. 7 is a perspective view in x-direction of the measuring device 40 of FIG. 4.

### References

- 1: Measuring unit
- 2: Support member
- 3: Upper bearing arm
- 4: Lower bearing arm
- 5: Base
- 6: Body
- 7: Recess
- 8: Upper fixing passage
- 9: Lower fixing passage
- 10: Shaft
- 11: Upper shaft toe
- 12: Lower shaft toe
- 13: Shaft body
- 14: Upper bearing unit
- 15: Fixing section
- 16: Bearing section
- 17: Adjusting means
- 18: Fixing means
- 19: Fixing passage
- 20: Rotation axis
- 21: Lower bearing unit
- 22: Collar
- 23: Bearing section
- 24: Interface member
- 25: Upper connector section
- 26: Frame
- 27: Lower connector section
- 28: Opening
- 29: Fixing unit
- 30: Front
- 31: Detecting element
- 32: Drive element
- 33: Receptacle
- 34: Fixing element
- 35, 35': Coupling shaft
- 36: Interface section
- 37: Probe connector section
- 38: Mounting element
- 39: Base plate
- 40: Measuring device
- 41: Detecting means
- 42: Probe element
- 43: Cup
- 44: Sample liquid
- 45: Spring
- 46: Movable bearing plate
- 47: Thrust bearing plate
- 48: Cup holder
- 49: Ball bearing
- 50: Groove
- 51: Circlip
- 52: Bearing cover plate

## Claims

1. A measuring unit (1) for measuring characteristics of a sample liquid (44), in particular viscoelastic characteristics of a blood sample, comprising
a support member (2) having at least one upper bearing arm (3) with an upper bearing unit (14), at least one lower bearing arm (4) with a lower bearing unit (21) and a base (5) for being attachable to a respective measuring system (40);
a shaft (10) having shaft toes (11, 12) and being rotatably supported about a rotation axis (20) by said upper bearing unit (14) and said lower bearing unit (21), wherein said upper bearing unit (14), said lower bearing unit (21) and said shaft toes (11, 12) form toe bearings, respectively;
an interface member (24) having a detecting element (31) and a drive element (32), said interface member (24) being fixed on said shaft (10) and being connected to a coupling shaft (35) with a probe connector section (37) for measuring characteristics of said sample liquid (44);
wherein said interface member (24) and the coupling shaft (35) are coaxially aligned with said shaft (10).

2. The measuring unit (1) according to claim 1, wherein said upper bearing unit (14) and said lower bearing unit (21) are removable inserts each being fixed in said respective bearing arm (3, 4).

3. The measuring unit (1) according to claim 1 or 2, wherein said lower bearing unit (21) is a thrust bearing and said upper bearing unit (14) is a moveable bearing being adjustable by adjusting means (17).

4. The measuring unit (1) according to at least one of the preceding claims, wherein said upper bearing unit (14) and said lower bearing unit (21) comprise bearing plates (46, 47) made of a kind of jewel and/or ceramic.

5. The measuring unit (1) according to at least one of the preceding claims, wherein said interface member (24) comprises:
an upper connector section (25) connected to said shaft (10);
a frame (26) having an opening (28) for a passage of said lower bearing arm (4) of said support member (2);
a lower connector section (27) connected to said coupling shaft (35);
a front (30) for securing that said detecting element (31) thereon; and
a receptacle (33) for securing that said drive element (32) therein.

6. The measuring unit (1) according to claim 5, wherein said interface member (24) further comprises a fixing unit (29) for fixing said interface member (24) to said shaft (10).

7. The measuring unit (1) according to claim 6, wherein said fixing unit (29) is at least one screw.

8. The measuring unit (1) according to claim 6, wherein said fixing unit (29) is formed having clip means cooperating with corresponding clip means of said shaft (10).

9. The measuring unit (1) according to at least one of the claims 5 to 8, wherein said lower connector section (27) is formed having clip means cooperating with corresponding clip means of an interface section (36) of said coupling shaft (35).

10. The measuring unit (1) according to at least one of the preceding claims, wherein said probe connector section (37) of said coupling shaft (35) is formed having clip means cooperating with corresponding clip means of a probe element being detachably fixed onto said probe connector section (37).

11. The measuring unit (1) according to at least one of the preceding claims, wherein said detecting element (31) is a mirror.

12. The measuring unit (1) according to at least one of the preceding claims, wherein the drive element (32) is a spring wire.

13. The measuring unit (1) according to at least one of the preceding claims, comprising axial stop means to limit an axial movement of the shaft (10) and the interface member (24).

14. The measuring unit (1) according to claim 13, wherein the axial stop means are formed by a shoulder of the interface member (24) or/and a shoulder of the shaft (10), said shoulder pointing to the upper bearing arm (3) and co-operating with a corresponding shoulder of the upper bearing arm (3) and/or the upper bearing unit (14).

15. The measuring unit (1) according to at least one of the preceding claims, wherein at least one of said upper bearing unit (14) and said lower bearing unit (21) comprises at least one bearing cover plate (52).

16. Measuring system (40) for measuring characteristics of a sample liquid, in particular viscoelastic characteristics of a blood sample, comprising at least one measuring unit (1) according to at least one of the preceding claims.

## Patentansprüche

1. Messeinheit (1) zur Messung von Eigenschaften einer Probenflüssigkeit (44), insbesondere von viskoelastischen Eigenschaften einer Blutprobe, umfassend:
ein Tragelement (2) mit mindestens einem oberen Lagerarm (3) mit einer oberen Lagereinheit (14), mindestens einem unteren Lagerarm (4) mit einer unteren Lagereinheit (21) und einer Basis (5) zur Anbringung an ein zugehöriges Messsystem (40);
eine Welle (10), welche mit Wellenspitzen (11, 12) versehen und durch die obere Lagereinheit (14) und die untere Lagereinheit (21) um eine Drehachse (20) drehbar gelagert ist, wobei die obere Lagereinheit (14), die untere Lagereinheit (21) und die Wellenspitzen (11, 12) jeweils Spitzenlager bilden;
ein Schnittstellenglied (24) mit einem Abtastelement (31) und einem Antriebselement (32), wobei das Schnittstellenglied (24) auf der Welle (10) befestigt und mit einer Kopplungswelle (35) mit einem Sondenverbindungsabschnitt (37) zur Messung von Eigenschaften der Probenflüssigkeit (44) verbunden ist;
wobei das Schnittstellenglied (24) und die Kopplungswelle (35) koaxial mit der Welle (10) ausgerichtet sind.

2. Messeinheit (1) nach Anspruch 1, wobei die obere Lagereinheit (14) und die untere Lagereinheit (21) entfernbare Einsätze sind, von denen jeder in dem zugehörigen Lagerarm (3, 4) befestigt ist.

3. Messeinheit (1) nach Anspruch 1 oder 2, wobei die untere Lagereinheit (21) ein Drucklager ist, und die obere Lagereinheit (14) ein verstellbares Lager ist, das durch Einstellmittel (17) einstellbar ist.

4. Messeinheit (1) nach mindestens einem der vorhergehenden Ansprüche, wobei die obere Lagereinheit (14) und die untere Lagereinheit (21) Lagerplatten (46, 47) aus einer Art Edelstein und/oder Keramik aufweisen.

5. Messeinheit (1) nach mindestens einem der vorhergehenden Ansprüche, wobei das Schnittstellenglied (24) umfasst:
einen oberen Verbindungsabschnitt (25), welcher mit der Welle (10) verbunden ist;
einen Rahmen (26) mit einer Öffnung (28) für einen Durchgang des unteren Lagerarms (4) des Tragelementes (2);
einen unteren Verbindungsabschnitt (27), welcher mit der Kopplungswelle (35) verbunden ist;
eine Vorderseite (30) zur Befestigung des Abtastelementes (31) darauf; und
eine Aufnahme (33) zur Befestigung des Antriebselementes (32) darin.

6. Messeinheit (1) nach Anspruch 5, wobei das Schnittstellenglied (24) weiterhin eine Befestigungseinheit (29) zur Befestigung des Schnittstellengliedes (24) an der Welle (10) aufweist.

7. Messeinheit (1) nach Anspruch 6, wobei die Befestigungseinheit (29) mindestens eine Schraube ist.

8. Messeinheit (1) nach Anspruch 6, wobei die Befestigungseinheit (29) mit einer Klipseinrichtung ausgebildet ist, welche mit einer korrespondierenden Klipseinrichtung der Welle (10) zusammenwirkt.

9. Messeinheit (1) nach mindestens einem der Ansprüche 5 bis 8, wobei der untere Verbindungsabschnitt (27) mit einer Klipseinrichtung ausgebildet ist, welche mit einer korrespondierenden Klipseinrichtung eines Anschlussabschnitts (36) der Kopplungswelle (35) zusammenwirkt.

10. Messeinheit (1) nach mindestens einem der vorhergehenden Ansprüche, wobei der Sondenverbindungsabschnitt (37) der Kopplungswelle (35) mit einer Klipseinrichtung ausgebildet ist, welche mit einer korrespondierenden Klipseinrichtung eines Sondenelementes zusammenwirkt, das auf dem Sondenverbindungsabschnitt (37) entfernbar befestigbar ist.

11. Messeinheit (1) nach mindestens einem der vorhergehenden Ansprüche, wobei das Abtastelement (31) ein Spiegel ist.

12. Messeinheit (1) nach mindestens einem der vorhergehenden Ansprüche, wobei das Antriebselement (32) ein Federdraht ist.

13. Messeinheit (1) nach mindestens einem der vorhergehenden Ansprüche, wobei sie axiale Arretiermittel zur Begrenzung einer axialen Verschiebung der Welle (10) und des Schnittstellengliedes (24) aufweist.

14. Messeinheit (1) nach Anspruch 13, wobei die axialen Arretiermittel durch eine Schulter des Schnittstellengliedes (24) oder/und durch eine Schulter der Welle (10) gebildet sind, wobei die Schulter zu dem oberen Lagerarm (3) weist und mit einer korrespondierenden Schulter des oberen Lagerarms (3) und/oder der oberen Lagereinheit (14) zusammenwirkt.

15. Messeinheit (1) nach mindestens einem der vorhergehenden Ansprüche, wobei mindestens eine von der oberen Lagereinheit (14) und der unteren Lagereinheit (21) mindestens eine Lagerdeckplatte (52) aufweist.

16. Messsystem (40) zur Messung von Eigenschaften einer Probenflüssigkeit, insbesondere von viskoelastischen Eigenschaften einer Blutprobe, umfassend mindestens eine Messeinheit (1) nach mindestens einem der vorhergehenden Ansprüche.

## Revendications

1. Unité de mesure (1) des caractéristiques d'un échantillon liquide (44), en particulier les propriétés visco-élastiques d'un échantillon sanguin, comprenant:
un élément de support (2) ayant au moins un bras supérieur de palier (3) avec une unité de palier (14) supérieure, au moins un bras de palier (4) inférieur avec une unité de palier (21) inférieure et une base (5) pour attache à un système de mesure (40) respectif;
un arbre (10) ayant des pointes de pivot (11, 12) et étant supporté pivotant sur un axe de rotation (20) par ladite unité de palier (14) supérieure et ladite unité de palier (21) inférieure, ladite unité de palier (14) supérieure, ladite unité de palier (21) inférieure et
lesdites pointes de pivot (11, 12) formant des suspensions à pivot respectives;
un élément de jonction (24) ayant un élément détecteur (31) et un élément d'actionnement (32), ledit élément de jonction (24) étant fixé audit arbre (10) et étant relié à un arbre d'accouplement (35) ayant une partie de raccordement (37) à un palpeur pour mesurer des caractéristiques dudit échantillon liquide (44);
ledit élément de jonction (24) et ledit arbre d'accouplement (35) étant alignés coaxialement sur ledit arbre (10).

2. Unité de mesure (1) selon la revendication 1, ladite unité de palier (14) supérieure et ladite unité de palier (21) inférieure sont formées comme dispositifs insérables amovibles, chaque étant fixée dans ledit bras de palier (3, 4) respectif.

3. Unité de mesure (1) selon la revendication 1 ou 2, ladite unité de palier (21) inférieure étant un coussinet de butée, et ladite unité de palier (14) supérieure étant un coussinet déplaçable et étant ajustable par des moyens d'ajustage (17).

4. Unité de mesure (1) selon l'une quelconque des revendications précédentes, ladite unité de palier (14) supérieure et ladite unité de palier (21) inférieure comprenant des plaques d'appui (46, 47) respectives fabriquées comme coussinet en rubis et/ou céramique.

5. Unité de mesure (1) selon l'une quelconque des revendications précédentes, ledit élément de jonction (24) comprenant:
une partie supérieure de raccordement (25) reliée audit arbre (10);
une cadre (26) avec une ouverture (28) pour un passage dudit bras de palier (4) inférieur dudit élément de support (2);
une partie inférieure de raccordement (27) reliée audit arbre d'accouplement (35);
une face (30) pour fixer ledit élément détecteur (31) là-dessus; et
un logement (33) pour fixer ledit élément d'actionnement (32) là-dedans.

6. Unité de mesure (1) selon la revendication 5, ledit élément de jonction (24) de plus comprenant un dispositif de fixation (29) pour fixer ledit élément de jonction (24) audit arbre (10).

7. Unité de mesure (1) selon la revendication 6, ledit dispositif de fixation (29) étant au moins une vis.

8. Unité de mesure (1) selon la revendication 6, ledit dispositif de fixation (29) étant formé avec des moyens d'une attache de type clip coopérant avec des moyens d'une attache de type clip correspondant dudit arbre (10).

9. Unité de mesure (1) selon l'une quelconque des revendications 5 à 8, ladite partie inférieure de raccordement (27) étant formée avec des moyens d'une attache de type clip coopérant avec des moyens d'une attache de type clip correspondant d'une partie de jonction (36) dudit arbre d'accouplement (35).

10. Unité de mesure (1) selon l'une quelconque des revendications précédentes, ladite partie de raccordement (37) à un palpeur dudit arbre d'accouplement (35) étant formée avec des moyens d'une attache de type clip coopérant avec des moyens d'une attache de type clip correspondant d'un élément palpeur étant fixé d'une façon amovible à ladite partie de raccordement (37) à un palpeur.

11. Unité de mesure (1) selon l'une quelconque des revendications précédentes, ledit élément détecteur (31) étant un miroir.

12. Unité de mesure (1) selon l'une quelconque des revendications précédentes, ledit élément d'actionnement (32) étant un fil d'acier pour ressort.

13. Unité de mesure (1) selon l'une quelconque des revendications précédentes, comprenant des moyens d'arrêt pour limiter un déplacement dudit arbre (10) et dudit élément de jonction (24).

14. Unité de mesure (1) selon la revendication 13, lesdits moyens d'arrêt étant formés par une épaule dudit élément de jonction (24) ou/et une épaule dudit arbre (10), ladite épaule se dirigeant vers ledit bras supérieur de palier (3) et coopérant avec une épaule correspondante dudit bras supérieur de palier (3) et/ou ladite unité de palier (14) supérieure.

15. Unité de mesure (1) selon l'une quelconque des revendications précédentes, au moins l'une de ladite unité de palier (14) supérieure et ladite unité de palier (21) inférieur comprenant au moins une plaque de recouvrement (52).

16. Système de mesure (40) pour mesurer des caractéristiques d'un échantillon liquide, en particulier les propriétés visco-élastiques d'un échantillon sanguin, comprenant au moins une unité de mesure (1) selon l'une quelconque des revendications précédentes.
